(19) Europäisches Patentamt
European Patent Office
Office européen des brevets

(11) **EP 3 496 668 B1**

(12) **FASCICULE DE BREVET EUROPEEN**

(45) Date de publication et mention de la délivrance du brevet:
**08.07.2020 Bulletin 2020/28**

(21) Numéro de dépôt: **17765230.2**

(22) Date de dépôt: **08.08.2017**

(51) Int Cl.:
***A61F 2/28*** (2006.01)

(86) Numéro de dépôt international:
**PCT/FR2017/052212**

(87) Numéro de publication internationale:
**WO 2018/029429 (15.02.2018 Gazette 2018/07)**

(54) **IMPLANT OSSEUX À BASE D'UN MATÉRIAU BIOACTIF**

KNOCHENIMPLANTAT AUF DER BASIS EINES BIOAKTIVEN MATERIALS

BONE IMPLANT BASED ON A BIOACTIVE MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.08.2016 FR 1657639**

(43) Date de publication de la demande:
**19.06.2019 Bulletin 2019/25**

(73) Titulaires:
• **Omnium De Revalorisation Industrielle ODRI**
**75116 Paris (FR)**
• **Odri, Guillaume-Anthony**
**75013 Paris (FR)**

(72) Inventeur: **ODRI, Guillaume-Anthony**
**75013 Paris (FR)**

(74) Mandataire: **Agasse, Stéphane et al**
**Cabinet GERMAIN & MAUREAU**
**B.P. 6153**
**69466 Lyon Cedex 06 (FR)**

(56) Documents cités:
**EP-A1- 0 369 034     WO-A2-2007/038559**
**US-A- 5 112 354      US-A1- 2002 123 750**

## Description

La présente invention concerne un implant osseux à base d'un matériau bioactif et qui est vascularisable dès l'implantation.

Par implant osseux à base d'un matériau bioactif, on entend un élément constitué d'un substitut osseux tel que défini par la Société française de recherche orthopédique et traumatologique (SOFROT), c'est-à-dire « tout biomatériau d'origine humaine, animale, végétale ou synthétique, 1) qui est destiné à l'implantation chez l'homme ; 2) dans la perspective d'une reconstitution du stock osseux ; et 3) par le renforcement d'une structure osseuse ou le comblement d'une perte de substance osseuse d'origine traumatique ou orthopédique ». On entend aussi, toute association d'un substitut osseux ainsi défini avec des moyens permettant de faciliter la mise en place de l'implant et/ou son intégration.

Le tissu osseux est l'organe qui structure et soutient le corps. Certaines pathologies entraînent une atteinte de la structure macroscopique des os et empêchent le tissu osseux d'assurer sa fonction de soutien architectural du corps : traumatismes avec fractures, pathologies tumorales, pathologies infectieuses, malformations congénitales. L'atteinte de la structure macroscopique de l'os peut aussi être voulue lors d'un geste chirurgical pour corriger une déformation, réséquer une pathologie tumorale ou créer un traumatisme osseux afin d'induire le phénomène de cicatrisation. La cicatrisation osseuse est un processus biologique spontané qui permet de restaurer l'intégrité architecturale de l'os atteint, que l'on appelle consolidation. Ce processus est néanmoins complexe et fragile et toute perturbation (radiothérapie, infection, perte de substance osseuse, ouverture cutanée, diabète, tabac...) entraine un ralentissement de la cicatrisation pouvant aboutir dans certains cas à une absence de consolidation, que l'on appelle pseudarthrose.

La chirurgie orthopédique utilise différentes techniques pour améliorer les chances de consolidation et limiter les séquelles. Elle aide le processus de cicatrisation osseuse à se dérouler dans de meilleures conditions. Cependant, le risque de pseudarthrose, toutes fractures confondues, reste élevé autour de 10%. Différentes techniques existent pour faire consolider un os qui n'a pas consolidé initialement, mais leur réalisation est complexe et onéreuse et nécessite des délais très longs pour des résultats variables. De plus, lorsqu'il existe une perte de substance osseuse critique, la consolidation ne sera jamais obtenue avec rétablissement de l'architecture initiale de l'os uniquement grâce au processus de cicatrisation physiologique, et un apport d'os, ou greffe osseuse, doit être réalisé. L'apport osseux peut se faire à partir d'os pris ailleurs sur le patient, notamment au niveau des crêtes iliaques, on parle d'autogreffe. Ce prélèvement osseux est très limité en quantité et n'est pas dénué de comorbidités. Sinon, il est possible de réaliser une greffe à partir d'os pris sur cadavre et traité de manière adéquate, on parle d'allogreffe. Cette technique présente le désavantage de ne pas aussi bien consolider que les autogreffes et nécessite de réaliser un prélèvement sur cadavre et de disposer d'une banque d'os.

De nombreuses recherches ont été réalisées afin de développer de l'os synthétique, mimant les caractéristiques de l'os normal. Après de nombreuses améliorations apportées aux biomatériaux initialement utilisés (en modifiant la composition chimique, la forme, l'architecture), les résultats obtenus sont toujours inférieurs à ceux obtenus avec l'os normal. Récemment, plusieurs facteurs biologiques ont été identifiés pouvant expliquer cette différence, en particulier l'absence de cellules souches mésenchymateuses, l'absence de facteurs de croissance et l'absence de vascularisation. De nombreux essais en laboratoire ont ainsi été réalisés pour apporter les facteurs biologiques manquant aux biomatériaux : apport de cellules souches mésenchymateuses, introduction de facteurs de croissance (BMP-2), co-cultures et impression 3D pour favoriser le développement de réseaux vasculaires. Cependant, les résultats expérimentaux *in vivo* ne sont pas à la hauteur des attentes. En effet, la plupart des techniques développées jusqu'à maintenant consistent à créer un implant présentant un environnement propice au développement de l'os et des vaisseaux. Mais, la plupart des cellules souches implantées meurent avant que l'implant soit envahi de nouveaux vaisseaux provenant des tissus environnants et cette absence de vascularisation précoce efficace semble être le facteur principal d'échec des biomatériaux actuels.

Le brevet WO 2007/038559 A2 montre un implant osseux à base d'un matériau bioactif et comportant des canaux facilitant l'intégration osseuse et/ou la résorption de l'implant.

De nombreuses recherches ont été réalisées et sont actuellement en cours pour développer un implant osseux vascularisable.

Les techniques mises en œuvre reposent sur les stratégies suivantes :

- Stimulation de l'angiogénèse par des facteurs de croissance (tels que VEGF, FGF) :
  Ces travaux révèlent une sensibilité majeure au type de facteur de croissance utilisé, à la dose, au mode et à la séquence d'administration du ou des facteurs de croissance, une moindre variation du protocole pouvant induire une inhibition de la résorption osseuse ;
- Activation du biomatériau avant sa transplantation, par co-cultures de cellules endothéliales et de cellules souches mésenchymateuses ;
- Stimulation mécanique de l'angiogénèse par application de contraintes de cisaillement favorisant la prolifération et la migration des cellules endothéliales ;
- Sélection de biomatériaux adaptés :
  Il existe de nombreux types de biomatériaux, mais les plus étudiés et utilisés sont les céramiques phosphocalciques qui présentent l'avantage d'être bioactives, c'est-à-dire qu'elles peuvent s'intégrer dans l'os sans présenter d'interface fibreux et sont capables d'ostéointégration. Parmi ces céramiques, les plus courantes sont l'hydroxyapatite, HAP de formule $Ca_{10}(PO_4)_6(OH)_2$, le phosphate tricalcique beta, βTCP de formule $Ca_3(PO_4)_2$, leurs dérivés et leurs combinaisons. Toutefois, l'HAP est très peu soluble et son taux de dégradation *in vivo* est assez bas. Le βTCP est beaucoup plus soluble et se dégrade bien *in vivo,* mais il est plus fragile. Les produits biphasés constitués de mélanges de telles céramiques, et en particulier ceux constitués à la fois de HAP et de βTCP ont des propriétés et un résultat clinique qui varient en fonction du rapport entre HAP et βTCP. Il ressort toutefois de ces études que la présence de pores de diamètres variables et connectés est essentielle à la survie des cellules souches mésenchymateuses et à l'envahissement du biomatériau par des néovaisseaux. Une revue complète de la littérature est faite dans un rapport de la Haute Autorité de la Santé (HAS) sur les substituts osseux en 2013 (http://www.has-sante.fr/portail/upload/docs/application/pdf/2013-06/rapport dévaluation des substituts osseux.pdf) (Haute Autorité de Santé. Substituts osseux. Révision de catégories homogènes de dispositifs médicaux. Saint-Denis La Plaine : HAS ; 2013) et conclut comme suit « L'hétérogénéité des données cliniques disponibles et le manque d'études comparatives prospectives rendent impossible l'identification d'un substitut osseux de référence parmi ceux évalués ».

- Microfabrication :

La microfabrication consiste à créer un réseau 3D précis de microcanaux, reproduisant un réseau vasculaire fonctionnel, prêts pour le développement de vaisseaux dans le biomatériau ou néovascularisation. Différents procédés peuvent être mis en œuvre comme l'electrospinning, l'extrusion bi-vis, la photolithographie, la microstéréolithographie...

- Préfabrication :

La préfabrication consiste à introduire les vaisseaux dans le biomatériau au moment de l'implantation, avec l'idée qu'une vascularisation à proximité du biomatériau peut servir de point de départ pour la néovascularisation de tissus. Elle consiste à réaliser une boucle artérioveineuse ou à implanter un pédicule vasculaire sous ou dans la greffe, résultant en un développement angiogénique spontané et une vascularisation de l'os à greffer. Parmi ces techniques, la boucle artério-veineuse est la plus efficace car on observe le développement d'un réseau capillaire perfusé qui se remodèle pour créer des artérioles, des veinules postcapillaires et des veinules.

Il s'avère qu'aucune de ces approches ne permet d'accéder à un apport de sang oxygéné et riche en nutriment au centre du greffon dès l'implantation. Tous ces dispositifs consistent à créer des conditions favorables à une néovascularisation, mais la survie des cellules souches implantées dans le biomatériau reste trop brève par rapport au développement d'une néovascularisation fonctionnelle.

Fort de ce constat et pour répondre à ce réel besoin d'un implant performant, l'inventeur a considéré que l'apport d'une vascularisation perfusant le greffon aussitôt l'implantation réalisée constituait un critère déterminant. Il a ainsi orienté ses recherches vers le développement d'un implant osseux configuré pour accueillir et maintenir, dès son implantation, le flux sanguin du système vasculaire de l'os traité.

L'invention fournit un implant qui rend possible un apport vasculaire dès l'implantation chez le patient et permet ainsi de lever nombre des obstacles auxquels les dispositifs connus se heurtent.

Ainsi, selon un premier aspect, l'invention porte sur un implant osseux à base d'un matériau bioactif, ledit implant possédant un réseau d'au moins deux canaux aptes à la perfusion sanguine, ledit réseau comprenant au moins un canal d'entrée de la perfusion présentant une ouverture ménagée dans la surface de l'implant et au moins un canal de sortie de la perfusion présentant une ouverture ménagée dans la surface de l'implant, les deux dits canaux allant du canal d'entrée au canal de sortie.

Par canal apte à la perfusion sanguine, on entend selon l'invention, un canal capable de recevoir, dès la mise en place de l'implant, le sang circulant du patient traité et d'en assurer la circulation, moyennant quoi le sang irrigue ledit implant par déplacement dans ledit réseau de canaux. Ainsi, parmi d'autres possibilités, un tel canal est apte à la perfusion sanguine s'il présente un diamètre sensiblement égal ou au moins égal à celui d'un vaisseau sanguin qui selon le site et le mode retenu d'implantation de l'implant, est généralement une artère. Son diamètre peut donc varier d'un à quelques dixièmes de millimètres jusqu'à quelques millimètres (de l'ordre d'une dizaine de millimètres).

Par rapport à l'ensemble des solutions proposées dans l'art antérieur qui ciblent une néovascularisation, c'est-à-dire la fabrication de nouveaux vaisseaux sanguins au sein de l'implant, un implant de l'invention est immédiatement vascularisable par le flux sanguin du patient. Bien entendu, un implant de l'invention peut être complété partout moyen favorisant sa néovascularisation.

Dans le présent texte, on entend par matériau bioactif pour un implant, un matériau naturel ou synthétique destiné à être implanté dans un organisme vivant et qui a la capacité d'accélérer les processus de réparation tissulaires et est amené, à cet effet, à interagir directement avec l'organisme, notamment à établir des liaisons chimiques avec le site d'implantation, par exemple avec l'os environnant. Un matériau bioactif est en outre biorésorbable, c'est-à-dire qu'il peut être absorbé et/ou dégradé par les tissus et/ou lessivé du site d'implantation et disparaître *in vivo* après un certain temps qui peut varier par exemple de quelques mois à quelques années, suivant la nature chimique du matériau et le métabolisme de l'organisme vivant. Les propriétés bioactives d'un matériau dépendent essentiellement de sa nature (structure chimique), de sa porosité, et de l'environnement du matériau.

Selon une mise en œuvre de l'invention, un matériau bioactif approprié répond aux normes en vigueur définissant les exigences applicables aux implants osseux. A titre d'exemples non limitatifs, un tel matériau est conforme aux normes internationales ISO 13175-3 ou ISO 13779-1; d'autres exemples sont donnés dans le rapport de l'HAS sur les substituts osseux, de mai 2013, précité.

Dans une forme de réalisation de l'invention, le matériau bioactif est une céramique phosphocalcique ou un mélange de céramiques phosphocalciques. Les céramiques phosphocalciques peuvent être d'origine biologique, fabriquées à partir de structures biologiques préexistantes (par exemple à partir de corail, d'os), ou d'origine synthétique. Ces dernières sont généralement produites à partir d'une poudre qui, après calcination, est compactée et soumise à un traitement de frittage, ce qui induit une fusion et l'agglomération en microcristaux qui se soudent entre eux, laissant des interstices d'une taille d'au plus 100 μm, d'environ 1 à 100 μm, de préférence de 1 à 50 μm, voire de 3 à 10 μm, ou micropores. Le matériau résultant est ensuite traité par un agent porogène, par exemple une poudre de billes de naphtalène, qui crée des macropores d'une taille d'au moins 100 μm, et de préférence d'une taille d'au plus 900 μm, de préférence d'au plus 700 μm, voire d'au plus 600 μm. La structure de la céramique obtenue ressemble à celle de la phase minérale de l'os, ce qui permet l'adsorption des protéines de la matrice osseuse par ledit matériau, et donc l'adhésion d'ostéoblastes et leur différenciation. Selon l'invention, le matériau bioactif est avantageusement choisi parmi l'hydroxyapatite (HAP), le phosphate tricalcique beta (βTCP), leurs dérivés et leurs combinaisons. Avantageusement, le matériau est une combinaison de HAP et de βTCP. En effet, la HAP étant très peu soluble dans les fluides biologiques, sa résorption est très lente et sa combinaison avec un autre matériau bioactif peut permettre de limiter cet inconvénient. Ainsi, les combinaisons de HAP et de βTCP, appelées aussi céramiques phosphocalciques biphasées (BCP), sont privilégiées. De telles céramiques sont disponibles dans le commerce ou peuvent être fabriquées à la demande. Il relève des compétences de l'homme du métier de choisir une céramique adaptée à l'implant à poser, comme par exemple de choisir une BCP, plus ou moins riche en HAP, le ratio HAP/βTCP (m/m) pouvant varier de 20/80 à 80/20, par exemple de 25/75 à 75/25, voire de 40/60 à 60/40.

Comme dit précédemment, la porosité est aussi un paramètre important dans les propriétés bioactives du matériau. Par porosité, on comprend principalement la distribution des pores, leur interconnectivité et leur taille.

La porosité du matériau bioactif de l'implant est de préférence une porosité ouverte. Ceci signifie que les pores du matériau sont reliés entre eux et forment un réseau de tubes capillaires, qui vont être pénétrés par des fluides, comme des liquides par exemple de l'eau, comme des gaz, par exemple de l'air, lesdits fluides étant éventuellement chargés de nutriments, de tout ou partie de molécules, par exemple biologiques, ou de cellules ou fragments de cellules, comme le sérum. La nature des éléments entrant dans ledit réseau de tubes capillaires dépend principalement de la taille des pores. Cette porosité est en particulier accessible aux molécules nécessaires à la survie des cellules fondatrices ou participant à la néovascularisation et l'ostéogénèse.

La porosité d'un matériau bioactif de l'invention est de préférence bi-modale, avec une microporosité et une macroporosité telles que définies précédemment.

De préférence, le volume des pores, représente en volume au moins 70%, mieux encore au moins 80%, voire plus, du volume du matériau bioactif.

Un implant tel que défini précédemment peut se présenter sous diverses formes et mises en œuvre, notamment eu égard à sa destination. Certaines formes de réalisation sont ci-après décrites, sans toutefois qu'un implant de l'invention y soit restreint.

Ainsi, dans une forme de réalisation de l'invention, le canal d'entrée, dit canal principal d'entrée, se sépare en au moins les deux canaux, dits canaux secondaires, les deux dits canaux secondaires se réunissant en le canal de sortie, dit canal principal de sortie.

Dans une autre forme de réalisation, l'implant comprend au moins deux canaux d'entrée et au moins deux canaux de sortie, pour respectivement chacun des deux canaux de l'implant aptes à la perfusion, dits canaux principaux. Selon cette variante, l'implant est irrigué par au moins deux canaux, chacun étant raccordé à son propre canal d'entrée et son propre canal de sortie.

Bien entendu, un implant de l'invention peut comporter un réseau de canaux comprenant les deux formes de réalisation ci-dessus.

Avantageusement, et afin de vasculariser au mieux l'implant de l'invention, le réseau d'au moins deux canaux aptes à la perfusion sanguine comporte une multiplicité dédits canaux aptes à la perfusion sanguine. Dans une forme préférée, les canaux dudit réseau sont interconnectés, au moins partiellement, c'est-à-dire que le réseau peut être assimilé à un maillage de canaux se recoupant en un ou plusieurs endroits de leur longueur. Une telle variante est illustrée plus loin dans la description.

Selon une variante optimisée d'un implant de l'invention et afin d'accroître le taux de réussite de l'implantation, la surface de l'implant est essentiellement étanche au sang, à l'exception desdites ouvertures du ou des canaux d'entrée et du ou des canaux de sortie qui y sont ménagées. Précisément, le sang irriguant l'implant ne peut pas sortir, ou fuiter, de l'implant, autrement que par le ou les canaux d'entrée/de sortie.

L'expression « essentiellement étanche au sang » signifie, dans le cadre de l'invention, que le sang ne fuit pas ou en tout cas le moins possible, en-dehors de l'implant, un suintement d'une fraction, notamment liquide, du sang, comme le sérum pouvant se produire.

Cette étanchéité constitue une barrière entre l'intérieur de l'implant et l'extérieur, et permet de créer un espace essentiellement clos dans lequel le sang pourra circuler depuis le ou les canaux d'entrée vers le ou les canaux de sortie, en favorisant une irrigation complète ou quasi complète dudit implant, sans fuir à l'extérieur autrement que par la ou les ouvertures du ou des canaux de sortie. Par une irrigation complète, on entend que l'ensemble du réseau de canaux dudit implant sont remplis du sang du patient.

La surface de l'implant peut être rendue étanche par tout moyen. A titre d'exemple, l'implant fabriqué à partir d'un matériau bioactif tel que défini précédemment peut être revêtu d'une couche d'un matériau bioactif tel que défini précédemment, de même nature ou de nature différente de celui de l'implant, à condition d'être hermétique au sang et de préférence à toute molécule favorisant la néovascularisation et l'ostéogénèse, afin d'assurer une vascularisation maximale dudit implant. Selon un autre exemple, l'implant est fabriqué d'une pièce, en un matériau bioactif qui est circonscrit par une couche de matériau bioactif hermétique au sang et de préférence à toute molécule favorisant la néovascularisation et l'ostéogénèse, comme peut le permettre une fabrication par impression 3D. Un tel matériau bioactif hermétique peut être un matériau bioactif poreux, à porosité au moins partiellement fermée. Avantageusement, au moins 50%, voire au moins 60% et même au moins 70%, des pores du matériau ne sont pas interconnectés.

Un implant de l'invention comporte un réseau de canaux, dont les paramètres suivants doivent être pris en compte pour assurer une vascularisation suffisante :

- Le diamètre des canaux,
- La forme du réseau,
- La distance entre deux canaux, et
- L'angle entre deux canaux connectés, c'est-à-dire que se recoupent.

Ces paramètres sont ci-après caractérisés.

- Diamètre des canaux

Comme dit précédemment, les canaux constituant le réseau sont de préférence de section constante. Ils peuvent avoir des diamètres identiques ou différents.

Pour assurer une reconstruction osseuse totale, l'écoulement doit se faire de manière aussi homogène que possible dans ledit implant et avec un certain débit qui sera prédéterminé en fonction du patient, de l'os substitué.... A minima, il doit se faire de manière à empêcher la thrombose qui bloquera tout afflux sanguin et donc ultérieurement les mécanismes biologiques nécessaires à la reconstruction osseuse.

Afin de comprendre les écoulements dans les différents canaux et de déterminer les dimensions des canaux selon l'invention, il convient d'utiliser, en première approximation, la loi de « Poiseuille » qui s'applique aux écoulements de « Poiseuille ». Le flux sanguin réel suit une loi plus complexe, mais cette approximation reste largement utilisée pour modéliser des écoulements dans des cylindres.

La loi de Poiseuille (également appelée loi de Hagen-Poiseuille) décrit l'écoulement laminaire d'un liquide visqueux dans une conduite cylindrique : pour être applicable le fluide doit donc être un fluide newtonien. Bien que le sang soit un fluide non newtonien, cette loi est largement utilisée en médecine pour expliquer les écoulements dans les vaisseaux sanguins, et dans la présente invention, elle est parfaitement applicable pour déterminer les écoulements dans les canaux d'un implant de l'invention.

Selon la loi de Poiseuille, le débit d'un liquide dans une conduite cylindrique est donné par la relation (1) :

$$Q = R_h \times \delta P$$

dans laquelle

Q représente le débit dans la conduite cylindrique (ou canal),
$\delta P$ représente la variation de pression dans la conduite cylindrique depuis l'entrée jusqu'à la sortie,
Rh représente la résistance hydraulique définie par la relation (2) :

$$R_h = \frac{8 \mu L}{\pi R^4}$$

dans laquelle

$\mu$ représente la viscosité dynamique du liquide,
L représente la longueur de la conduite cylindrique, et
R représente le rayon de la conduite cylindrique.

Ainsi, selon l'invention, les vitesses d'écoulement, ou débits Q, du sang, dans les canaux peuvent être différentes, selon les dimensions, longueur et rayon, des canaux, mais il est préférable d'obtenir un débit globalement homogène. Ainsi, les canaux de longueurs plus petites pourront présenter un rayon inférieur à celui des canaux de longueurs plus grandes. A partir des relations (1) et (2) précitées de la loi de Poiseuille, l'homme du métier pourra adapter le diamètre de chaque canal afin que l'écoulement se fasse de la manière la plus homogène possible en fonction de la longueur du canal. En particulier, pour obtenir un débit prédéterminé, plus le canal sera long, plus son rayon devra être important. La détermination des dimensions des canaux sera optimisée pour limiter le risque de thrombose du patient tout en favorisant la reconstruction osseuse. Il ressort des compétences générales de l'homme du métier d'effectuer cette optimisation.

A titre d'illustration, par application de la loi de Poiseuille, le débit d'écoulement sanguin dans chaque canal d'un implant de l'invention, lesdits canaux ayant le même rayon et la même longueur, sera égal au débit du canal principal d'entrée dudit implant divisé par le nombre de canaux.

Comme indiqué précédemment, les canaux possèdent un diamètre qui peut aller d'environ 0,1 mm jusqu'à environ 15 mm, généralement d'environ 0,1 mm à environ 10 mm, selon le type de vaisseau sanguin à raccorder audit implant. Dans sa version la plus largement utilisée, un implant de l'invention sera irrigué par le sang d'une artère et le diamètre des canaux sera de préférence d'au moins 0,1 mm et/ou de préférence d'au plus 5 mm. Le réseau est avantageusement constitué de canaux de diamètre variable entre des canaux de faible diamètre, des canaux de diamètre moyen et des canaux de diamètre plus grand.

- La forme du réseau

Selon l'invention, le réseau est constitué d'au moins deux canaux; il peut comporter une multiplicité de canaux. Quel que soit leur nombre, les dimensions des canaux peuvent être identiques ou différentes, mais toujours déterminées aux fins d'une vascularisation optimale et d'une limitation des risques circulatoires.

Comme dit précédemment, les canaux du réseau sont avantageusement interconnectés, totalement ou au moins partiellement. La forme du réseau est prédéterminée en fonction de la forme de l'implant en vue d'une irrigation optimale. Le réseau peut être fabriqué par micro-usinage. Le réseau de canaux pourra être constitué de canaux principaux directement connectés au(x) vaisseau(x) sanguin(s) du patient, respectivement au niveau des canaux d'entrée et de sortie, au moyen des angiocathéters. Il pourra être complété par des canaux secondaires directement connectés aux canaux principaux. Ce réseau pourra aussi comporter des canaux tertiaires reliés auxdits canaux secondaires, et pourra être de même être doté de canaux quaternaires reliés auxdits canaux tertiaires, et ainsi de suite.

- Position des canaux

La position des canaux doit être déterminée de manière à ce que les canaux ne soient pas trop proches au risque de fragiliser l'implant; toutefois, les canaux interconnectés, ne doivent pas être trop éloignés, sous peine de ne pas offrir une perfusion optimale dans les canaux et ainsi ne pas permettre une reconstruction osseuse totale.

- Angles entre deux canaux connectés

Une règle générale pour ce type d'écoulement est d'éviter tout angle trop fort, de type angle droit, lorsque deux canaux se recoupant forment un angle. Plus l'angle est progressif, meilleur sera l'écoulement dans les canaux connectés. Ainsi, un angle d'au plus 60 degrés est souhaitable. Ce paramètre doit donc être pris en compte dans la fabrication d'un implant de l'invention.

L'ensemble des paramètres ci-dessus sont prédéterminés en fonction de l'usage de l'implant.

Ainsi, bien qu'un implant de l'invention soit adapté à tout type de reconstruction osseuse, l'invention concerne aussi une mise en œuvre particulière de l'implant, lorsque celui-ci est destiné à relier deux parties d'un os tubulaire.

Selon cette variante, l'implant est constitué d'un corps longitudinal, de deux extrémités dans chacune desquelles au moins une ouverture est ménagée, d'un conduit médullaire s'étendant le long de l'axe longitudinal dudit implant, depuis une dite extrémité à l'autre et débouchant dans chacune desdites ouvertures, et d'un réseau d'au moins deux canaux aptes à la perfusion sanguine, ledit réseau comprenant au moins un canal d'entrée de la perfusion présentant une ouverture ménagée dans la surface de l'implant et au moins un canal de sortie de la perfusion présentant une ouverture ménagée dans la surface de l'implant, les deux dits canaux allant du canal d'entrée au canal de sortie. Chacune des ouvertures ménagées dans les extrémités de l'implant est avantageusement équipée d'un élément en forme de cône tronqué ouvert, ou cône médullaire, fixé sur les faces basales du corps longitudinal, par sa plus grande base pour relier l'implant au conduit médullaire de l'os, et ainsi augmenter la stabilité mécanique de l'implant dans l'os. Dans cette configuration, le conduit médullaire de l'implant sera initialement comblé par du sang de la moelle osseuse de l'os adjacent, puis par de la moelle osseuse. Ceci permettra d'avoir un remodelage osseux d'origine médullaire afin de reconstituer la structure architecturale normale de l'os avec corticales et médullaire.

Dans cette forme particulière, les ouvertures du ou des canaux d'entrée et de sortie sont avantageusement formées dans la surface du corps longitudinal de l'implant.

Le réseau de canaux peut consister en au moins deux et de préférence une multiplicité de canaux interconnectés, dont certains s'étendent approximativement le long de l'axe longitudinal dudit implant et les autres traversent ceux-ci, selon un axe qui est de préférence non strictement perpendiculaire. Comme décrit ci-dessus, le réseau est constitué de canaux principaux, et peut comprend des canaux secondaires, puis tertiaires, voire plus.

Afin d'optimiser la vascularisation aussi complète que possible le conduit médullaire de l'implant, dans une variante de l'invention, est essentiellement étanche au sang, afin d'éviter toute perte du sang d'irrigation dans ledit conduit. Les conditions d'étanchéité du conduit médullaire sont identiques à celles de la surface de l'implant, décrites précédemment. Selon cette variante, le réseau de canaux ne traverse de préférence pas le conduit médullaire afin d'éviter toute perte de sang dans ledit conduit, alors l'implant comprend au moins un canal et de préférence un réseau de canaux interconnectés de part et d'autre du conduit médullaire, chacun de canaux ou de ces réseaux étant relié à au moins une ouverture pour le canal d'entrée et une ouverture pour le canal de sortie ménagées dans le corps longitudinal de l'implant.

Tout implant selon l'invention, peut selon un aspect particulier de l'invention, comporter sur chacune des ouvertures du ou des canaux d'entrée et du ou des canaux de sortie un angiocathéter. Les angiocathéters permettent de connecter un vaisseau sanguin audit implant, ce qui va permettre d'apporter une vascularisation riche en oxygène et nutriments. Il appartient aux connaissances générales de l'homme du métier de choisir des angiocathéters appropriés, fournis dans le commerce. Avantageusement, ils sont fixés au moyen d'une colle chirurgicale connue de l'homme du métier, par exemple de type Bioglue® de chez Gamida ou Tissucol® de chez Baxter.

Un autre objet de l'invention consiste en un procédé de fabrication d'un implant de l'invention, étanche, tel que décrit précédemment. Le procédé ci-après défini est adapté à la réalisation d'un implant de l'invention quelle que soit sa destination, et donc quels que soient sa forme, sa taille et son réseau de canaux interconnectés.

Le procédé comprend les étapes suivantes :

- On dispose d'un matériau bioactif,
- On revêt toute la surface de l'implant d'un matériau bioactif étanche,
- On réalise au moins un canal d'entrée et au moins un canal de sortie,
- On usine un réseau de canaux interconnectés, par toute technique bien connue de l'homme du métier et notamment par des techniques de micro-usinage pouvant faire intervenir le tournage, le fraisage, le perçage et/ou la découpe numérique ou manuelle, et
- On revêt les orifices laissés par l'usinage d'un matériau bioactif étanche, à l'exception des canaux d'entrée et de sortie.

Selon une autre variante de l'invention, un implant de l'invention peut être fabriqué par impression 3D.

A l'issue du procédé, l'implant ainsi fabriqué devra être nettoyé et stérilisé selon des techniques classiques bien connues de l'homme du métier.

En plus de l'intérêt majeur d'un implant de l'invention lié à sa vascularisation immédiate dès son implantation chez le patient, l'implant ne nécessite aucun traitement préalable, notamment aucun traitement *in vitro* auquel nécessite de recourir certains implants connus.

Ainsi, un autre objet de l'invention réside dans un procédé d'implantation d'un implant tel que décrit précédemment.

La procédure est réalisée en condition de microchirurgie avec utilisation d'un microscope (par exemple de type Leica).

La technique chirurgicale habituellement utilisée par le chirurgien pour aborder la zone à opérer doit être suivie. Lorsque la zone d'implantation du greffon est exposée, il faut exciser le tissu d'interposition entre les deux extrémités osseuses. Il peut être nécessaire de faire une recoupe osseuse afin de créer un espace correspondant à la longueur du greffon.

La diaphyse est préparée à l'aide d'un ancillaire spécifique permettant de préparer les corticales afin que :

- Elles s'interrompent à angle droit,
- Un chanfrein interne soit préparé pour une meilleure congruence entre l'implant et l'os au niveau des cônes médullaires.

L'implant doit s'introduire à frottement dur dans la diaphyse. Les raccords entre les corticales et l'implant sont scellés à l'aide de colle chirurgicale, de manière à les rendre hermétiques. Ceci évite une fuite sanguine par l'interface.

Les raccords vasculaires sont ensuite réalisés. La circulation peut alors être lancée, et il faut colmater les zones d'extravasation vasculaire.

L'ostéosynthèse est ensuite réalisée de manière classique, de type plaque vissée à l'aide de vis verrouillées.

Selon une variante de cette procédure, des facteurs de croissance peuvent être ajoutés à l'implant : soit des facteurs pro-angiogéniques (VEGF), soit des facteurs ostéoinducteurs (BMP2), soit une association des deux.

L'invention sera mieux comprise, grâce à la description ci-après, qui se rapporte à des modes de réalisation préférés, donnés à titre d'exemples non limitatifs, et expliqués en référence aux figures annexées présentées ci-après :

La figure 1 montre un schéma en coupe transversale d'un implant de l'invention destiné à relier deux parties d'un os tubulaire.
La figure 2 scindée en quatre sous figures 2A, 2B, 2C et 2D sont des vues en 3D d'un implant de l'invention le montrant dans son ensemble selon la figure 2A, puis en coupes longitudinales selon les figures 2B et 2C respectivement perpendiculaires l'une par rapport à l'autre, et en coupe transversale selon la figure 2D.
La figure 3 illustre une reconstitution en 3D du conduit médullaire et des canaux principaux et secondaires d'un implant illustré à la figure 2.

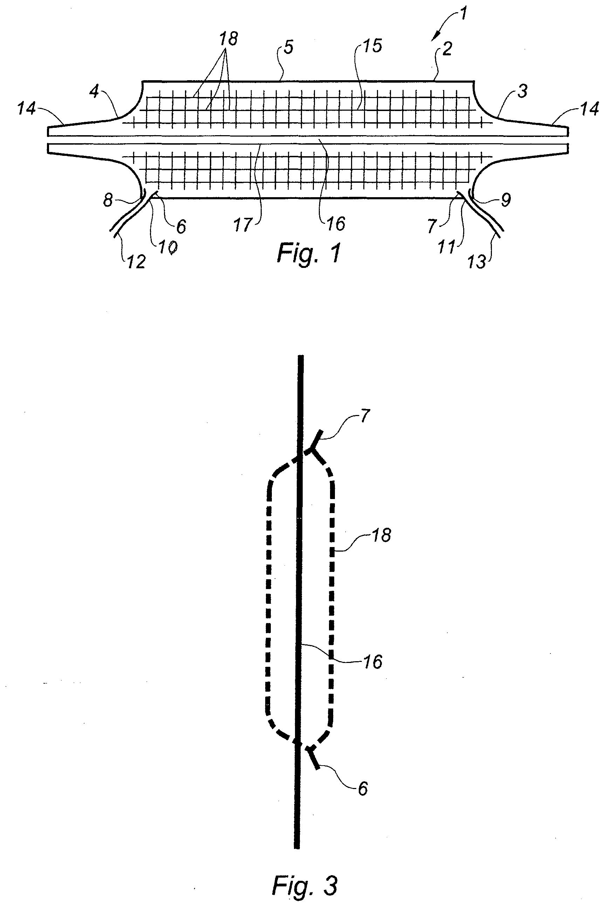

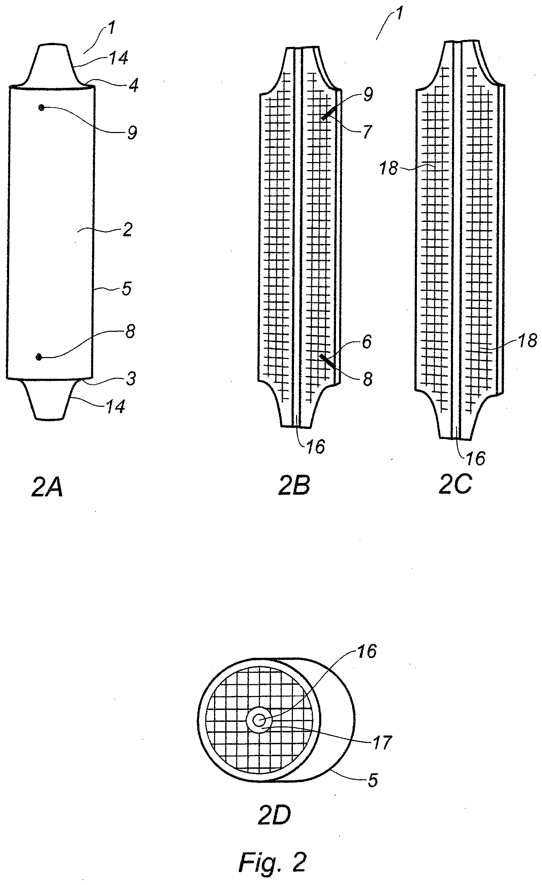

Un implant osseux 1 comporte un corps cylindrique 2 et deux extrémités 3 et 4. Le diamètre du corps cylindrique est déterminé pour correspondre à celui de l'os traité. L'implant dont les vues font l'objet des figures 2 a un diamètre de 3 cm et une longueur de 10 cm. L'implant a une surface 5 essentiellement étanche au sang et dans laquelle sont définis un canal principal d'entrée 6 et un canal principal de sortie 7, au moyen des ouvertures 8 et 9 qui y sont ménagées. Le canal d'entrée 6 et le canal de sortie 7 sont chacun équipés d'un angiocathéter, 10 et 11, respectivement, par l'intermédiaire desquels le canal d'entrée 6 est relié au vaisseau sanguin afférent 12 et le canal de sortie 7 est relié au vaisseau sanguin efférent 13. Les deux extrémités 3 et 4 de l'implant 1 sont prolongées en cônes médullaires 14. Ces cônes s'introduisent dans les extrémités diaphysaires de l'os à combler, permettant ainsi d'augmenter la surface de contact entre l'implant et l'os à combler afin d'améliorer les chances d'ostéointégration et la tenue primaire de l'implant. L'implant 1 est en un matériau bioactif 15 poreux et comporte un conduit médullaire central 16 s'étendant le long de l'axe longitudinal du corps cylindrique 2, depuis l'extrémité 3 à l'extrémité 4 et se prolongeant dans les cônes 14. Ce conduit permet la diffusion de cellules ostéoprogénitrices et la vascularisation à partir de la moelle osseuse de l'os comblé. La surface 17 du conduit médullaire central 16 est essentiellement étanche au sang qui irrigue ledit implant 1. L'implant 1 comporte un réseau de canaux définis dans ledit matériau bioactif 15 qui comprend une multiplicité de canaux secondaires 18, s'étendant le long de l'axe longitudinal du corps cylindrique 2. Le canal principal d'entrée 6 et le canal principal de sortie 7 pénètrent l'implant selon un angle variable. Ils traversent les canaux secondaires 18 de plus faibles calibres et passent de part et d'autre du conduit médullaire central 16 sans communiquer avec celui-ci comme il ressort des figures 2D et 3. Les ouvertures 8 et 9 sont donc reliées entre elles par ce réseau de canaux. C'est au moyen de ces ouvertures qu'un cathéter sera branché sur une artère afin d'assurer la vascularisation de l'implant. Ces canaux communiquent avec le réseau de pores au sein du dispositif, illustrés aux figures 2B, 2C et 2D, afin de permettre une vascularisation de l'ensemble de l'implant.

Sur la figure 3, on observe que le conduit central n'est pas en communication avec les canaux principaux et secondaires. Le canal principal 6 pénètre l'implant selon un angle qui peut varier d'un implant à l'autre, puis se divise en 2 canaux secondaires 18 qui s'incurvent de manière à se prolonger dans l'axe longitudinal de l'implant et s'incurvent à nouveau en sens inverse pour se rejoindre au niveau du canal principal 7 inséré dans l'implant selon un angle qui peut varier d'un implant à l'autre.

Dès l'implantation, l'implant sera irrigué du sang du patient entrant dans le canal principal d'entrée 6, se répandant dans les canaux secondaires 18 et circulant jusqu'au canal principal de sortie 7 pour retourner dans le système vasculaire du patient. Le corps de l'implant est revêtu d'une couche de biomatériau qui dans le cas de l'implant de la figure 2 est de 2 mm, ce biomatériau étant hermétique pour empêcher la fuite du sang apporté à l'implant par les canaux reliés par cathéters au sang artériel.

## Revendications

1. Implant osseux (1) à base d'un matériau bioactif (15) et possédant un réseau d'au moins deux canaux (18) aptes à la perfusion sanguine, ledit réseau comprenant au moins un canal d'entrée (6) de la perfusion présentant une ouverture (8) ménagée dans la surface (5) de l'implant (1) et au moins un canal de sortie (7) de la perfusion présentant une ouverture (9) ménagée dans la surface (5) de l'implant (1), **caractérisé en ce que** chacun des deux dits canaux (18) vont du canal d'entrée (6) au canal de sortie (7).

2. Implant selon la revendication 1, **caractérisé en ce que** le canal d'entrée (6), dit canal principal d'entrée, se sépare en au moins les deux canaux (18), dits canaux secondaires, les deux dits canaux secondaires se réunissant en le canal de sortie (7), dit canal principal de sortie.

3. Implant selon la revendication 1, **caractérisé en ce qu'**il comprend au moins deux canaux d'entrée (6) et au moins deux canaux de sortie (7), pour respectivement chacun des deux canaux, dits canaux principaux.

4. Implant selon l'une quelconque des revendications précédentes, **caractérisé en ce que** le réseau d'au moins deux canaux (18) aptes à la perfusion sanguine comporte une multiplicité (18) dédits canaux aptes à la perfusion sanguine.

5. Implant selon l'une quelconque des revendications précédentes, **caractérisé en ce que** la surface (5) de l'implant (1) est en un matériau essentiellement étanche au sang, à l'exception desdites ouvertures (8, 9) du ou des canaux d'entrée (6) et du ou des canaux de sortie (7) qui y sont ménagées.

6. Implant selon l'une quelconque des revendications précédentes, **caractérisé en ce que** la surface (5) dudit implant (1) est rendue étanche par une couche en un matériau bioactif choisi parmi les céramiques phosphocalciques poreuses, à porosité au moins partiellement fermée.

7. Implant selon l'une quelconque des revendications précédentes, **caractérisé en ce que** le matériau bioactif (15) dudit implant (1) est choisi parmi les céramiques phosphocalciques poreuses, à porosité ouverte, et en particulier les hydroxyapatites, les phosphates tricalciques et leurs combinaisons.

8. Implant selon la revendication 6 ou 7, **caractérisé en ce que** le matériau bioactif (15) dudit implant (1) ou ledit matériau bioactif étanche est une céramique phosphocalcique biphasée qui comprend une hydroxyapatite et un phosphate tricalcique beta.

9. Implant selon l'une quelconque des revendications précédentes, **caractérisé en ce que** le matériau bioactif (15) de l'implant (1) présente une porosité ouverte, dont le volume représente au moins 70% du volume du matériau bioactif.

10. Implant selon l'une quelconque des revendications précédentes, **caractérisé en ce que** le matériau bioactif (15) de l'implant (1) présente une porosité ouverte, bi-modale, comprenant une microporosité avec des tailles de pores d'au plus 100 μm et une macroporosité avec des tailles de pores d'au moins 100 μm et d'au plus 900 μm.

11. Implant selon l'une quelconque des revendications précédentes, **caractérisé en ce que** lesdits deux canaux (18) au moins ont des sections constantes, identiques ou différentes, et possèdent un diamètre d'au moins 0,1 mm et d'au plus 10 mm.

12. Implant selon l'une quelconque des revendications 1 à 11, **caractérisé en ce que**, sur chacune des ouvertures (8,9) du ou des canaux d'entrée (6) et du ou des canaux de sortie (7), un angiocathéter (10, 11) est fixé.

13. Implant selon la revendication 12, **caractérisé en ce que** ledit angiocathéter (10, 11) est collé au moyen d'une colle chirurgicale.

14. Implant selon l'une quelconque des revendications précédentes, **caractérisé en ce qu'**il est destiné à relier deux parties d'un os tubulaire, et est constitué d'un corps longitudinal (2), de deux extrémités (3, 4) dans chacune desquelles au moins une ouverture est ménagée, et d'un conduit médullaire (16) s'étendant le long de l'axe longitudinal dudit implant (1), depuis une dite extrémité (3) à l'autre (4) et débouchant dans chacune desdites ouvertures.

15. Implant selon la revendication 14, **caractérisé en ce que** les ouvertures (8, 9) du ou des canaux d'entrée (6) et du ou des canaux de sortie (7) sont ménagées dans la surface (5) du corps longitudinal (2) de l'implant (1).

16. Implant selon la revendication 14 ou 15, **caractérisé**

**en ce que**, de part et d'autre du conduit médullaire (16), il comprend au moins un canal d'entrée (6), au moins un canal de sortie (7), et au moins un canal (18) allant dudit canal d'entrée audit canal de sortie.

17. Implant selon l'une quelconque des revendications 14 à 16, **caractérisé en ce que** le conduit médullaire (16) est un matériau essentiellement étanche au sang.

18. Implant selon l'une quelconque des revendications 14 à 17, **caractérisé en ce qu'**un élément en forme de cône tronqué ouvert (14), ou cône médullaire, est fixé, par sa grande base, sur chacune des faces basales du corps longitudinal de l'implant.

19. Procédé de fabrication d'un implant selon l'une quelconque des revendications 1 à 18, **caractérisé en ce qu'**il comprend les étapes suivantes :

- On dispose d'un matériau bioactif (15),
- On revêt toute la surface de l'implant d'un matériau bioactif étanche,
- On réalise au moins un canal d'entrée (6) et au moins un canal de sortie (7),
- On usine un réseau de canaux interconnectés, et
- On revêt les orifices laissés par l'usinage d'un matériau bioactif étanche, à l'exception des canaux d'entrée (6) et de sortie (7).

**Patentansprüche**

1. Knochenimplantat (1) auf Basis eines bioaktiven Materials (15) und ein Netz aus mindestens zwei Kanälen (18) besitzend, die für die Durchblutung geeignet sind, wobei das Netz mindestens einen Eintrittskanal (6) der Durchblutung umfasst, der eine Öffnung (8) aufweist, die in der Oberfläche (5) des Implantats (1) ausgestaltet ist, und mindestens einen Austrittskanal (7) der Durchblutung, der eine Öffnung (9) aufweist, die in der Oberfläche (5) des Implantats (1) ausgestaltet ist, **dadurch gekennzeichnet, dass** jeder der zwei genannten Kanäle (18) vom Eintrittskanal (6) zum Austrittskanal (7) verläuft.

2. Implantat nach Anspruch 1, **dadurch gekennzeichnet, dass** sich der Eintrittskanal (6), Haupteintrittskanal genannt, in mindestens die zwei Kanäle (18), Sekundärkanäle genannt, trennt, wobei sich die zwei sogenannten Sekundärkanäle zum Austrittskanal (7), Hauptaustrittskanal genannt, vereinigen.

3. Implantat nach Anspruch 1, **dadurch gekennzeichnet, dass** es für jeweils jeden der zwei Kanäle, Hauptkanäle genannt, mindestens zwei Eintrittskanäle (6) und mindestens zwei Austrittskanäle (7) umfasst.

**4.** Implantat nach einem der vorstehenden Ansprüche, **dadurch gekennzeichnet, dass** das Netz aus mindestens zwei Kanälen (18), die für die Durchblutung geeignet sind, eine Vielzahl (18) der für die Durchblutung geeigneten Kanäle umfasst.

**5.** Implantat nach einem der vorstehenden Ansprüche, **dadurch gekennzeichnet, dass** die Oberfläche (5) des Implantats (1) aus einem Material ist, das mit Ausnahme der Öffnungen (8, 9) des oder der Eintrittskanäle (6) und des oder der Austrittskanäle (7), die darin ausgestaltet sind, im Wesentlichen für Blut undurchlässig ist.

**6.** Implantat nach einem der vorstehenden Ansprüche, **dadurch gekennzeichnet, dass** die Oberfläche (5) des Implantats (1) durch eine Schicht aus einem bioaktiven Material undurchlässig gemacht wird, das aus den porösen Calciumphosphat-Keramiken mit mindestens teilweise geschlossener Porosität ausgewählt ist.

**7.** Implantat nach einem der vorstehenden Ansprüche, **dadurch gekennzeichnet, dass** das bioaktive Material (15) des Implantats (1) aus den porösen Calciumphosphat-Keramiken mit offener Porosität, und insbesondere den Hydroxyapatiten, den Tricalciumphosphaten und deren Kombinationen ausgewählt ist.

**8.** Implantat nach Anspruch 6 oder 7, **dadurch gekennzeichnet, dass** es sich bei dem bioaktiven Material (15) des Implantats (1) oder dem undurchlässigen bioaktiven Material um eine zweiphasige Calciumphosphat-Keramik handelt, die einen Hydroxyapatit und ein Beta-Tricalciumphosphat umfasst.

**9.** Implantat nach einem der vorstehenden Ansprüche, **dadurch gekennzeichnet, dass** das bioaktive Material (15) des Implantats (1) eine offene Porosität aufweist, deren Volumen mindestens 70 % des Volumens des bioaktiven Materials darstellt.

**10.** Implantat nach einem der vorstehenden Ansprüche, **dadurch gekennzeichnet, dass** das bioaktive Material (15) des Implantats (1) eine offene, bimodale Porosität aufweist, die eine Mikroporosität mit Porengrößen von höchsten 100 $\mu$m, und eine Makroporosität mit Porengrößen von mindestens 100 $\mu$m und höchstens 900 $\mu$m umfasst.

**11.** Implantat nach einem der vorstehenden Ansprüche, **dadurch gekennzeichnet, dass** mindestens die zwei Kanäle (18) konstante Querschnitte aufweisen, die gleich sind oder sich unterscheiden, und einen Durchmesser von mindestens 0,1 mm und höchstens 10 mm besitzen.

**12.** Implantat nach einem der Ansprüche 1 bis 11, **dadurch gekennzeichnet, dass** an jeder der Öffnungen (8, 9) des oder der Eintrittskanäle (6) und des oder der Austrittskanäle (7) ein Angiokatheter (10, 11) befestigt ist.

**13.** Implantat nach Anspruch 12, **dadurch gekennzeichnet, dass** der Angiokatheter (10, 11) mittels eines chirurgischen Klebers angeklebt ist.

**14.** Implantat nach einem der vorstehenden Ansprüche, **dadurch gekennzeichnet, dass** es dafür bestimmt ist, zwei Teile eines Röhrenknochens zu verbinden, und aus einem länglichen Körper (2), zwei Enden (3, 4), in jedem von denen mindestens eine Öffnung ausgestaltet ist, und einer Knochenmarkröhre (16) besteht, die sich entlang der Längsachse des Implantats (1) von einem genannten Ende (3) zum anderen (4) erstreckt und in jeder der Öffnungen mündet.

**15.** Implantat nach Anspruch 14, **dadurch gekennzeichnet, dass** die Öffnungen (8, 9) des oder der Eintrittskanäle (6) und des oder der Austrittskanäle (7) in der Oberfläche (5) des länglichen Körpers (2) des Implantats (1) ausgestaltet sind.

**16.** Implantat nach Anspruch 14 oder 15, **dadurch gekennzeichnet, dass** es auf beiden Seiten der Knochenmarkröhre (16) mindestens einen Eintrittskanal (16), mindestens einen Austrittskanal (7), und mindestens einen Kanal (18) umfasst, der vom Eintrittskanal zum Austrittskanal verläuft.

**17.** Implantat nach einem der Ansprüche 14 bis 16, **dadurch gekennzeichnet, dass** es sich bei der Knochenmarkröhre (16) um ein Material handelt, das im Wesentlichen für Blut undurchlässig ist.

**18.** Implantat nach einem der Ansprüche 14 bis 17, **dadurch gekennzeichnet, dass** ein Element in Form eines offenen Kegelstumpfs (14), oder Knochenmarkkegel, mit seiner großen Basis an jeder der Grundflächen des länglichen Körpers des Implantats befestigt ist.

**19.** Verfahren zur Herstellung eines Implantats nach einem der Ansprüche 1 bis 18, **dadurch gekennzeichnet, dass** es die folgenden Schritte umfasst:

- Verfügen über ein bioaktives Material (15),
- Beschichten der gesamten Oberfläche des Implantats mit einem undurchlässigen bioaktiven Material,
- Ausführen von mindestens einem Eintrittskanal (6) und mindestens einem Austrittskanal (7),

- Herausarbeiten eines Netzes von miteinander verbundenen Kanälen, und
- Beschichten der Löcher, die durch das Herausarbeiten hinterlassen wurden, mit einem undurchlässigen bioaktiven Material, mit Ausnahme der Einritts- (6) und Austrittskanäle (7).

**Claims**

1. A bone implant (1) based on a bioactive material (15) and having a network of at least two channels (18) capable of blood perfusion, said network comprising at least one input channel (6) of the perfusion having an aperture (8) formed in the surface (5) of the implant (1) and at least one output channel (7) of the perfusion having an aperture (9) formed in the surface (5) of the implant (1), **characterized in that** each of said two channels (18) goes from the input channel (6) to the output channel (7).

2. The implant according to claim 1, **characterized in that** the input channel (6), called main input channel, is separated into at least the two channels (18), called secondary channels, the said two secondary channels are joined in the output channel (7), called main output channel.

3. The implant according to claim 1, **characterized in that** it comprises at least two input channels (6) and at least two output channels (7), respectively for each of the two channels, called main channels.

4. The implant according to any one of the preceding claims, **characterized in that** the network of at least two channels (18) capable of blood perfusion includes a multiplicity (18) of said channels capable of blood perfusion.

5. The implant according to any one of the preceding claims, **characterized in that** the surface (5) of the implant (1) is essentially made of a blood-tight material, except for said apertures (8, 9) of the input channel(s) (6) and the output channel(s) (7) formed therein.

6. The implant according to any one of the preceding claims, **characterized in that** the surface (5) of said implant (1) is sealed by a layer made of a bioactive material selected from porous calcium phosphate ceramics, with at least partially closed porosity.

7. The implant according to any one of the preceding claims, **characterized in that** the bioactive material (15) of said implant (1) is selected from porous calcium phosphate ceramics, with open porosity, and particularly hydroxyapatites, tricalcium phosphates and combinations thereof.

8. The implant according to claim 6 or 7, **characterized in that** the bioactive material (15) of said implant (1) or said sealed bioactive material is a two-phase calcium phosphate ceramic which comprises a hydroxyapatite and a beta tricalcium phosphate.

9. The implant according to any one of the preceding claims, **characterized in that** the bioactive material (15) of the implant (1) has an open porosity, whose volume represents at least 70% of the volume of the bioactive material.

10. The implant according to any one of the preceding claims, **characterized in that** the bioactive material (15) of the implant (1) has a bi-modal open porosity, comprising a microporosity with pore sizes of at most 100 $\mu$m and a macroporosity with pore sizes of at least 100 $\mu$m and at most 900 $\mu$m.

11. The implant according to any one of the preceding claims, **characterized in that** said two channels (18) at least have constant, identical or different sections, and have a diameter of at least 0.1 mm and at most 10 mm.

12. The implant according to any one of claims 1 to 11, **characterized in that**, on each of the apertures (8, 9) of the input channel(s) (6) and the output channel(s) (7), an angiographic catheter (10, 11) is fixed.

13. The implant according to claim 12, **characterized in that** said angiographic catheter (10, 11) is glued by means of a surgical glue.

14. The implant according to any one of the preceding claims, **characterized in that** it is intended to connect two portions of a tubular bone, and is constituted of a longitudinal body (2), two ends (3, 4) in each of which at least one aperture is formed, and a medullary conduit (16) extending along the longitudinal axis of said implant (1), from a said end (3) to the other (4) and opening into each of said apertures.

15. The implant according to claim 14, **characterized in that** the apertures (8, 9) of the input channel(s) (6) and the output channel(s) (7) are formed in the surface (5) of the longitudinal body (2) of the implant (1).

16. The implant according to claim 14 or 15, **characterized in that**, on either side of the medullary conduit (16), it comprises at least one input channel (6), at least one output channel (7), and at least one channel (18) going from said input channel to said output channel.

17. The implant according to any one of claims 14 to 16, **characterized in that** the medullary conduit (16) is essentially a blood-tight material.

**18.** The implant according to any one of claims 14 to 17, **characterized in that** an element in the shape of an open truncated cone (14), or a medullary cone, is fixed, by its large base, on each of the basal faces of the longitudinal body of the implant.

**19.** A method for manufacturing an implant according to any one of claims 1 to 18, **characterized in that** it comprises the following steps:

- Disposing a bioactive material (15),
- Coating the entire surface of the implant with a sealed bioactive material,
- Producing at least one input channel (6) and at least one output channel (7),
- Manufacturing a network of interconnected channels, and
- Coating the orifices left by machining a sealed bioactive material, except for the input (6) and output (7) channels.

Fig. 1

Fig. 3

2A        2B        2C

2D

Fig. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007038559 A2